# EUROPEAN PATENT APPLICATION

(11) **EP 4 663 242 A1**
(43) Date of publication of application: **17.12.2025**
(21) Application number: 24181431.8
(22) Date of filing: 11.06.2024
(51) Int. Cl.: A61P 31/04, C07D 231/12, C07D 263/32, C07D 413/04

(54) **TARGETING THE ENERGY COUPLING FACTOR (ECF) TRANSPORTERS AS NOVEL ANTIMICROBIAL TARGET**

(71) Applicant: Helmholtz-Zentrum für Infektionsforschung GmbH, 38124 Braunschweig (DE); Goethe-Universität Frankfurt am Main, 60629 Frankfurt am Main (DE)
(72) Inventor: HAMED, Mostafa, Braunschweig (DE); HIRSCH, Anna K.H., Braunschweig (DE); WILLOCX, Daan, Braunschweig (DE); KANY, Andreas, Braunschweig (DE); HAUPENTHAL, Jörg, Braunschweig (DE); PROSCHAK, Ewgenij, Frankfurt am Main (DE); LILLICH, Felix, Frankfurt am Main (DE)
(74) Representative: Forstmeyer, Dietmar

(57) **Abstract**

The present invention relates to novel inhibitors of the Energy Coupling Factor (ECF) Transporters. These compounds of general formula (Ia) or (Ib) are useful in the treatment of bacterial infections, especially caused by *Streptococcus pneumoniae, Enterococcus faecium, Enterococcus faecalis.*

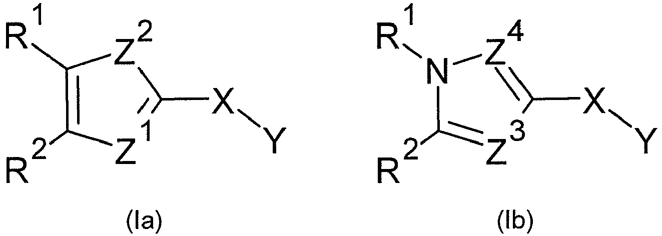

## Description

The present invention relates to novel inhibitors of the Energy Coupling Factor (ECF) Transporters. These compounds are useful in the treatment of bacterial infections, especially caused by *Streptococcus pneumoniae, Enterococcus faecium, Enterococcus faecalis.*

The energy-coupling factor (ECF) transporters are a family of transmembrane proteins involved in the uptake of vitamins into a wide range of bacteria. The term ECF transporter was coined in the 1970s to describe transporters that are used in *Lactobacillus casei* for the uptake of vitamins.¹ Later on, ECF transporters have been found to be particulary abundant in the Firmicutes phylum of Gram-positive bacteria, including human pathogenic species.² Importantly, they are not present in eukaryotes and are specific importers of essential micronutrients such as water-soluble vitamins (folate³, riboflavin⁴, cobalamin⁵, biotin⁶, niacin⁷, thiamine⁸, pantothenate⁹) and metal cations (Ni²⁺ and Co²⁺)¹⁰ into bacteria and archaea. Clinically relevant bacteria where the ECF tranporters genes are essential are: *S*. *pneumoniae, Staphylococcus aureus, E. faecium, E. faecalis, Clostridium tetani, C. novyi* and *C*. *difficile*.¹¹ Therefore, ECF transporters can be considered as novel potential antimicrobial targets to tackle infection due to their central role in the metabolism of bacteria.

They belong to the adenosine 5'-triphosphate (ATP)-binding cassette (ABC) transporters and consist of two ATP-hydrolyzing, nucleotide-binding domain named ECF-A and, two transmembrane proteins, termed ECF-T and the S-component.^{12, 13} The ECF-A and ECF-T together form the tricomponent ECF module.¹³ Main scaffold of the entire complex is the integral membrane ECF-T having a coupling domain on the cytoplasmatic side and two long α-helices that are arranged in X shape. These α-helices interact tightly with the ATPases and move jointly when, upon nucleotide binding and release, the ATPases switch between the open and closed conformation. The S-components are integral membrane proteins (20-25 KDa) and bind the substrate to be transported. This organization is typical and a unique feature for the ECF transporters, as other ABC utilize a soluble substrate-binding protein to capture ligands.¹⁴

ECF transporters are classified into two groups, group-I and -II. The ECF module in group-I interacts exclusively with a single "dedicated" S-component, whereas the module in group-II interacts with different ones. The present invention describes novel inhibitors of the ECF-type II, meaning that multiple S-components (specific for different substrate) can interact with the same ECF module,¹⁵ opening up the possibility to simultaneously block the uptake of several vitamins at the same time with only one inhibitor.

The present invention provides compounds of formula (Ia) and (Ib): 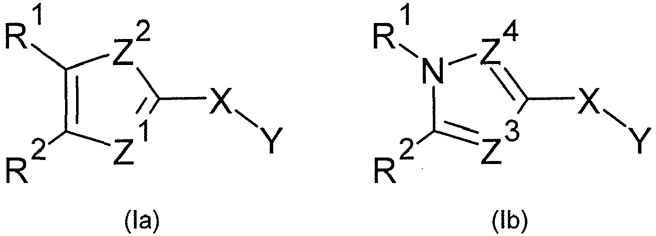 wherein
Z¹ N or CH;
Z² is O, S or NH;
Z³ is N or CH;
Z⁴ is N or CH;
Y is hydrogen; halogen; -C₁₋₄ alkyl; -C₁₋₄ haloalkyl; COOH; OH; CONH₂; CONHR'; PO(OH)₂; or PO(OR')₂;
R' is a C₁₋₄ alkyl group;
X is a bond; a C₁₋₄ alkylene group; a -S-C₁₋₄ alkylene group; a -O-C₁₋₄ alkylene group; a -NH-C₁₋₄ alkylene group; an optionally substituted phenylene group; or an optionally substituted heteroarylene group containing 5 or 6 ring atoms that are independently selected from C, N, O and S;
R¹ is hydrogen, halogen, an alkyl group, a cycloalkyl group, an optionally substituted phenyl group; or an optionally substituted heteroaryl group containing 5 or 6 ring atoms that are independently selected from C, N, O and S; and
R² is hydrogen, halogen, an alkyl group, a cycloalkyl group, an optionally substituted phenyl group; or an optionally substituted heteroaryl group containing 5 or 6 ring atoms that are independently selected from C, N, O and S;
or a salt thereof.

According to a preferred embodiment, the present invention provides compounds of formula (Ila) and (IIb): 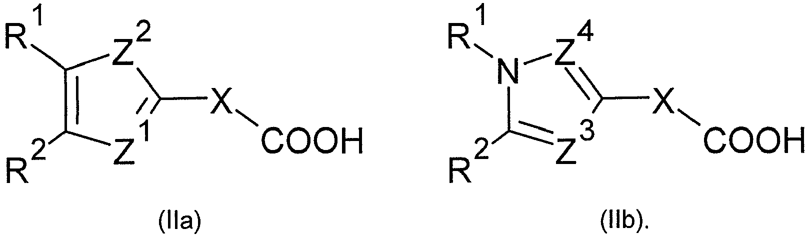 wherein Z¹, Z², Z³, Z⁴, R¹, R² and X are as defined above or below, or a salt thereof.

According to a further preferred embodiment, the present invention provides compounds of formula (III): 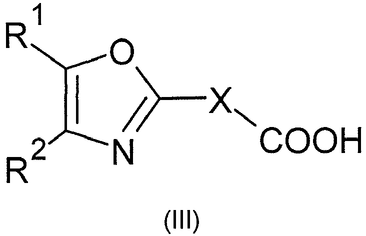 wherein R¹, R² and X are as defined above or below, or a salt thereof.

Preferably, X is a C₁₋₄ alkylene group; an optionally substituted phenylene group; or an optionally substituted heteroarylene group containing 5 or 6 ring atoms that are independently selected from C, N, O and S;

Further preferably, R¹ is an optionally substituted phenyl group; or an optionally substituted heteroaryl group containing 5 or 6 ring atoms that are independently selected from C, N, O and S; and

Moreover preferably, R² is an optionally substituted phenyl group; or an optionally substituted heteroaryl group containing 5 or 6 ring atoms that are independently selected from C, N, O and S;

Further preferably, X is selected from the following groups:

-CH₂-CH₂-; -CH₂-CH₂-CH₂-;

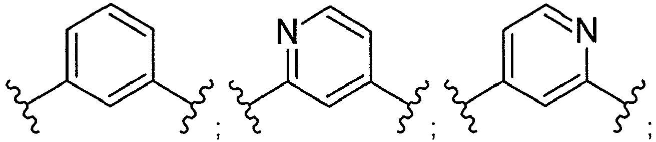

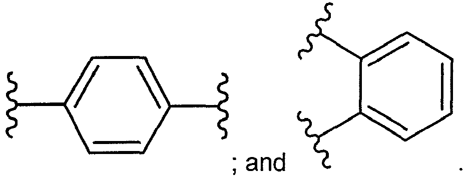

Further preferably, X is a bond or selected from the following groups: -S-CH(CH₃)-; - CH₂CH₂-; and -CH₂-.

Further preferably, X is selected from the following groups:

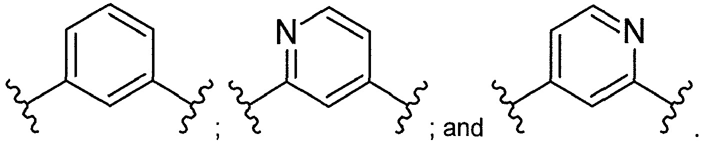

Moreover preferably, X is the following group:

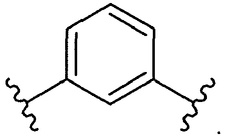

Further preferably, R¹ is an optionally substituted phenyl group.

Moreover preferably, R² is an optionally substituted phenyl group.

The term "optionally substituted" refers to a group which is unsubstituted or substituted by one or more (especially by one, or two; preferably by one) substituent(s).

If a group comprises more than one substituent, these substituents are independently selected, i.e., they may be the same or different.

Preferably, the optional substituents are independently selected from halogen; -OH; - NH₂; -CN; -C₁₋₄ alkyl; -C₁₋₄ haloalkyl; -O-C₁₋₄ alkyl; -O-C₁₋₄ haloalkyl; -SO₂NH₂; - SO₂CH₃; -O-CH₂-CH₂-; and -O-CH₂-O-.

Further preferably, the optional substituents are independently selected from -F; -Cl; - Me; -OMe; -CF₃; -OCF₃; -SO₂NH₂; -SO₂CH₃; -OCH(CH₃)₂; and -CN.

According to a preferred embodiment of the present invention, the compounds disclosed in the following publications as such are excluded from the present invention:
1) Ehrler JHM, Brunst S, Tjaden A, Kilu W, Heering J, Hernandez-Olmos V, Krommes A, Kramer JS, Steinhilber D, Schubert-Zsilavecz M, Müller S, Merk D, Proschak E. "Compilation and evaluation of a fatty acid mimetics screening library". Biochem Pharmacol. 2022 Oct; 204:115191. doi: 10.1016/j.bcp.2022.115191. Epub 2022 Jul 27. PMID: 35907497; and
2) Kramer JS, Woltersdorf S, Duflot T, Hiesinger K, Lillich FF, Knöll F, Wittmann SK, Klingler FM, Brunst S, Chaikuad A, Morisseau C, Hammock BD, Buccellati C, Sala A, Rovati GE, Leuillier M, Fraineau S, Rondeaux J, Hernandez-Olmos V, Heering J, Merk D, Pogoryelov D, Steinhilber D, Knapp S, Bellien J, Proschak E. "Discovery of the First in Vivo Active Inhibitors of the Soluble Epoxide Hydrolase Phosphatase Domain". J Med Chem. 2019 Sep 26; 62(18): 8443-8460. doi: 10.1021/acs.jmedchem.9b00445. Epub 2019 Sep 17. PMID: 31436984; PMCID: PMC7262789.

According to another embodiment, the use of these compounds as inhibitors of the Energy Coupling Factor (ECF) Transporters is encompassed by the present invention; especially the use of these compounds in the treatment of bacterial infections, especially caused by *Streptococcus pneumoniae, Enterococcus faecium, Enterococcus faecalis.*

The most preferred compounds of the present invention are the compounds disclosed in the examples, or a salt thereof.

It is further preferred to combine the preferred embodiments of the present invention in any desired manner (e.g., any embodiment for R¹ may be combined with any embodiment of R²).

The expression alkyl refers to a saturated, straight-chain or branched hydrocarbon group that contains from 1 to 20 carbon atoms, preferably from 1 to 15 carbon atoms, especially from 1 to 10 (e.g., 1, 2, 3 or 4) carbon atoms, for example a methyl (Me, CH₃), ethyl (Et), *n*-propyl (*n*Pr), *iso*-propyl (*i*Pr), *n*-butyl (*n*Bu), *iso*-butyl (*i*Bu), *sec*-butyl (*s*Bu), *tert*-butyl (*t*Bu), *n*-pentyl, *iso*-pentyl, *n*-hexyl, 2,2-dimethylbutyl or *n*-octyl group.

The expression C₁₋₄ alkyl refers to a saturated, straight-chain or branched hydrocarbon group that contains from 1 to 4 carbon atoms; for example, a methyl (Me, CH₃), ethyl (Et), *n*-propyl (*n*Pr), *iso*-propyl (*i*Pr), *n*-butyl (*n*Bu), *iso-*butyl (*i*Bu), *sec*-butyl (*s*Bu), or *tert*-butyl (*t*Bu) group.

The expression C₁₋₄ haloalkyl refers to a saturated, straight-chain or branched hydrocarbon group that contains from 1 to 4 carbon atoms, wherein one or more hydrogen atoms have been replaced by a halogen atom (preferably F or CI); for example, a 2,2,2-trichloroethyl or a trifluoromethyl (CF₃) group.

The expression cycloalkyl refers to a saturated or partially unsaturated (for example, a cycloalkenyl group) cyclic group that contains one or more rings (preferably 1 or 2), and contains from 3 to 14 ring carbon atoms, preferably from 3 to 10 (especially 3, 4, 5, 6 or 7) ring carbon atoms. Specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, propellane (e.g., [1.1.1]propellane) tetraline, cyclopentylcyclohexyl, or cyclohex-2-enyl group. Preferably, the expression cycloalkyl refers to a saturated cyclic group that contains one ring and from 3 to 7 ring carbon atoms.

Examples for a heteroaryl group containing 5 or 6 ring atoms that are independently selected from C, N, O and S are pyridyl, imidazolyl, thiazolyl, isothiazolyl, 1,2,3-triazolyl, 1,2,4-triazolyl, oxadiazolyl, thiadiazolyl, pyrazinyl, pyrimidinyl, pyridazinyl, oxazolyl, tetrazolyl, and isoxazolyl groups.

The suffix "-ene" like e.g., in "phenylene" refers to the corresponding divalent group.

The term halogen refers to F, Cl, Br or I.

Owing to their substitution, the compounds of the present invention may contain one or more centers of chirality. The present invention therefore includes both all pure enantiomers and all pure diastereomers and also mixtures thereof in any mixing ratio. The present invention moreover also includes all *cis*/*trans-*isomers of the compounds of the present invention and also mixtures thereof. The present invention moreover includes all tautomeric forms of the compounds of the present invention.

The present invention further provides pharmaceutical compositions comprising one or more compounds described herein or a salt, solvate or hydrate thereof, optionally in combination with one or more carrier substances and/or one or more adjuvants. The pharmaceutical composition of the present invention may contain a further antibacterial compound.

The present invention furthermore provides compounds or pharmaceutical compositions as described herein for use in the treatment of bacterial infections, especially caused by *Streptococcus pneumoniae, Enterococcus faecium, Enterococcus faecalis.*

The present invention further provides a compound as described herein or a pharmaceutical composition as defined herein for the preparation of a medicament for the treatment of bacterial infections, especially caused by *Streptococcus pneumoniae, Enterococcus faecium, Enterococcus faecalis.*

Examples of salts (especially of pharmacologically acceptable salts) of sufficiently basic compounds are salts of physiologically acceptable mineral acids like hydrochloric, hydrobromic, sulfuric and phosphoric acid; or salts of organic acids like methanesulfonic, *p*-toluenesulfonic, lactic, acetic, trifluoroacetic, citric, succinic, fumaric, maleic and salicylic acid. Further, a sufficiently acidic compound may form alkali or earth alkali metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts; all of which are also further examples of salts of the compounds described herein.

The compounds described herein may be solvated, especially hydrated. The solvation/ hydration may occur during the process of production or as a consequence of the hygroscopic nature of the initially water-free compounds. The solvates and/or hydrates may e.g. be present in solid or liquid form.

The therapeutic use of the compounds described herein, their salts, solvates and hydrates, respectively, as well as formulations and pharmaceutical compositions also lie within the scope of the present invention.

In general, the compounds and pharmaceutical compositions described herein will be administered by using the established and acceptable modes known in the art.

For oral administration, such therapeutically useful agents can be administered by one of the following routes: oral, e.g. as tablets, dragees, coated tablets, pills, semisolids, soft or hard capsules, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystals or as a spray (e.g., liquid aerosol), transdermal, for example via an transdermal drug delivery system (TDDS) such as a plaster containing the active ingredient or intranasal. For the production of such tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, and the like. For the production of soft capsules, one may use excipients as are e.g., vegetable, petroleum, animal or synthetic oils, wax, fat, and polyols. For the production of liquid solutions, emulsions or suspensions or syrups one may use as excipients e.g., water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. For aerosol formulations, one may use compressed gases suitable for this purpose, e.g., oxygen, nitrogen and carbon dioxide. The pharmaceutically useful agents may also contain additives for conservation, stabilization, e.g., UV stabilizers, emulsifiers, sweetener, aromatizers, salts to change the osmotic pressure, buffers, coating additives and antioxidants.

In general, in the case of oral or parenteral administration to adult humans weighing approximately 80 kg, a daily dosage of about 1 mg to about 10,000 mg, preferably from about 5 mg to about 1,000 mg, should be appropriate, although the upper limit may be exceeded when indicated. The daily dosage can be administered as a single dose or in divided doses, or for parenteral administration, it may be given as continuous infusion or subcutaneous injection.

### EXAMPLES

### 1. Chemistry

All compounds were synthesized via two previously reported synthetic routes (Schemes 1 and 2).^{16,17} The first route starts from the respective 1,2-diphenylethan-1-one (I), either synthesized *via* a previously published method¹⁶ or commercially obtained, which was then brominated in the presence of Br₂ (II).

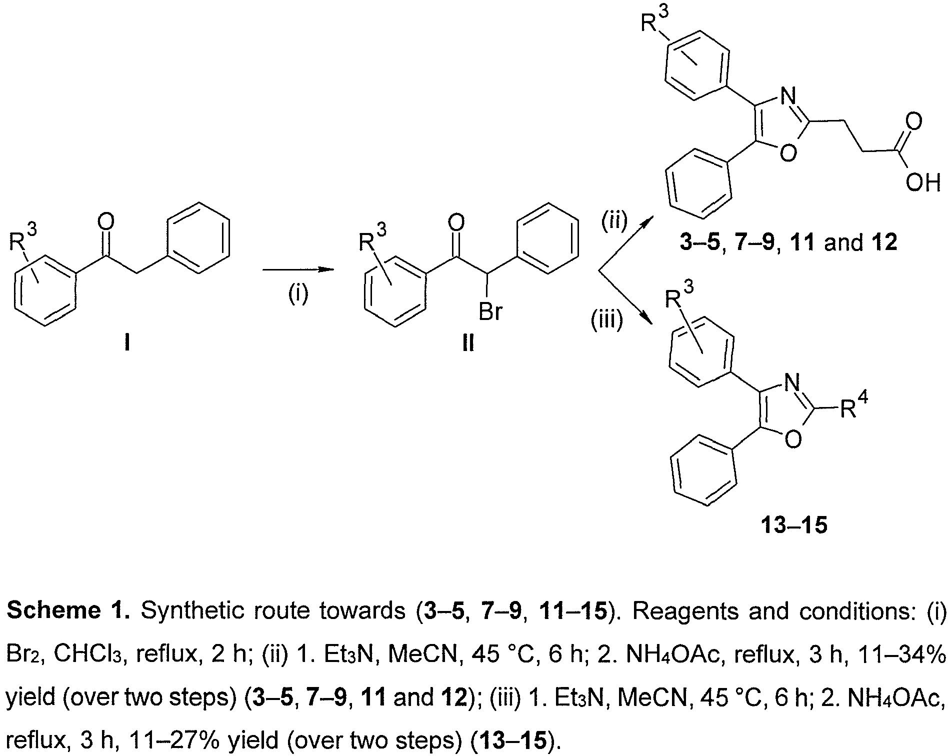

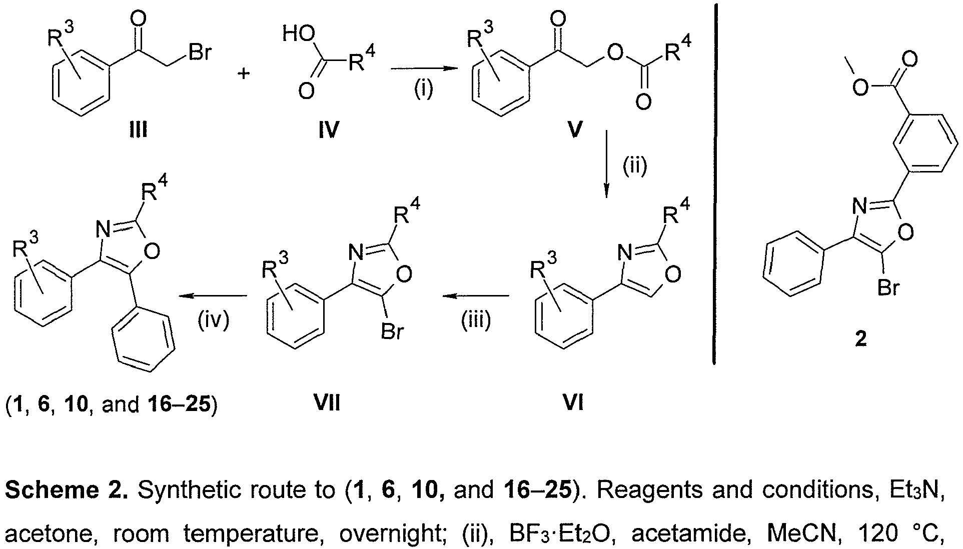

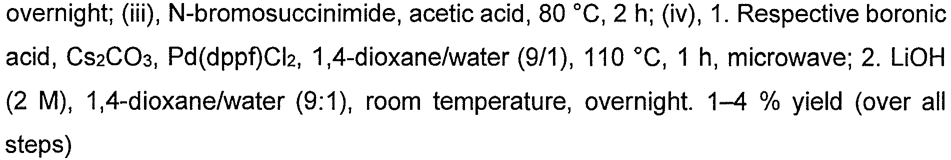

The resulting brominated intermediate was then mixed, without prior purification, with the respective carboxylic acid and the mixture was heated to 45 °C for 6 h and refluxed for 3 h to form the oxazole and afford the final product (Scheme 1). The second synthetic route commences from the respective 2-bromo-1-phenylethan-1-one (**III**), which was added to a basic solution containing the corresponding carboxylic acid **IV**, affording the related 2-oxo-2-phenylethyl acetate (**V**). Heating this intermediate to 120 °C in a solution containing BF₃·Et₂O and acetamide generated the oxazole **VI**, which was selectively brominated at the 5-position (**VII**) using N-bromosuccinimide (NBS). Lastly, a Suzuki reaction with phenylboronic acid and subsequent saponification of a potential ester ultimately provided the desired compound (Scheme 2).

### 2. SAR

Compounds **1** and **3-12** (Table 1) were synthesized to evaluate the structure-activity relationship at R². Analysis of compounds **1** and **3-6** revealed a preference for halogen atoms at this position of the phenyl ring. Comparable activities to **1** were obtained for fluoro- **(3)** and trifluoromethyl-substituted **(4)** derivatives, while derivatives bearing non-halogenated moieties **(5, 6)** demonstrated decreased activity. Next, the influence of the position of the halogen on the phenyl ring **(7-9)** was investigated. It became clear that both *para* **(1, 3)** and *meta* **(7)** positions are favorable for retaining activity, whereas substitution in the *ortho* position led to a decrease in activity **(8, 9).** Further, disubstitution led to an increase in potency, with **12** (ECF-T %inhibition at 50 µM = 42.9 ± 12.3) demonstrating double the inhibition at 50 µM) compared to the parent compound **1** (ECF-T %inhibition at 50 µM = 21.1 ± 0.7).

**Table 1. In vitro activities for compounds (1, 3-12) in ECF-T whole-cell uptake assay**

| 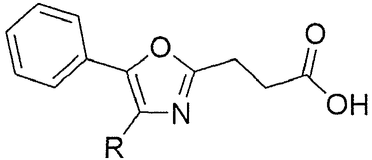 | | | | | |
|---|---|---|---|---|---|
| **#** | Structure, R= | % inhibition at 50 µM^{a} | **#** | Structure, R= | % inhibition at 50 µM^{a} |
| **1** | 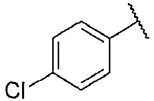 | 21.1 ± 0.7 | **8** | 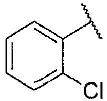 | 7.5 ± 1.7 |
| **3** | 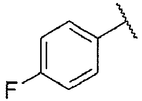 | 23.8 ± 1.5 | **9** | 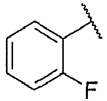 | 11 ± 1.6 |
| **4** | 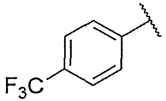 | 26.2 ± 0.9 | **10** | 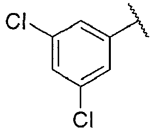 | 36.6 ± 1.1 |
| **5** | 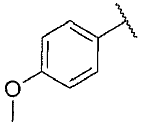 | 12.1 ± 4.2 | **11** | 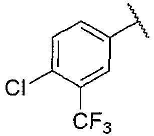 | 32.6 ± 12.7 |
| **6** | 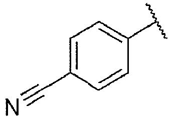 | 7.7 ± 0.3 | **12** | 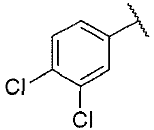 | 42.9 ± 12.3 |
| **7** | 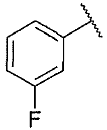 | 25.4 ± 2.5 | | | |

| | | | | | |
|---|---|---|---|---|---|
| ^{a}Assays were performed in replicate as independent experiments (n = 2); values are shown as mean ± SD. | | | | | |

In order to evaluate the structure-activity relationship at group X, **12** was used as starting point. At first, the linker length was increased by one carbon atom **(13),** which led to a decrease in activity. Since this decrease in activity might be due to the increased flexibility of the alkyl linker, it was replaced with a more rigid phenyl ring **(14-16)** and the carboxylic acid was arranged in all possible orientations. Here the *meta-*substituted derivative demonstrated superior activity over the other orientations and the parent compound **(12).** To further capitalize on the increase in potency by this replacement, the phenyl was interchanged with a pyridine ring to increase the hydrophilicity. This exchange led to a slight decrease in activity **(15,** ECF-T IC₅₀ = 5.1 ± 2.6 µM; **17** ECF-T IC₅₀ = 8.2 ± 0.9 µM).

**Table 2. In vitro activities for compounds (13-17) in ECF-T whole-cell uptake assay**

| 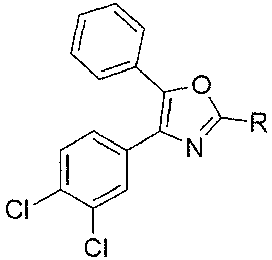 | | | |
|---|---|---|---|
| # | Structure, R= | % inhibition at 50 µM | IC₅₀ (µM) |
| **13** | 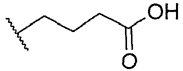 | 28 ± 3.1 | n.d. |
| **14** | 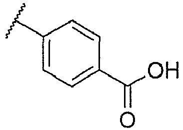 | 35.6 ± 1.5 | n.d. |
| **15** | 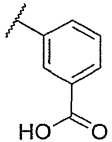 | 94.8 ± 3.6 | 5.1 ± 2.6 |
| **16** | 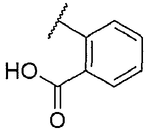 | 83.4 | n.d. |
| **17** | 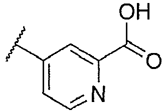 | 99.6 ± 0.1 | 8.2 ± 0.9 |

| | | | |
|---|---|---|---|
| ^{a}Assays were performed in replicate as independent experiments (*n* ≥ 2); values are shown as mean ± SD; n.d. = not determined. | | | |

In order to evaluate the structure-activity relationship at R¹, **15** was selected as starting point. When analyzing the following results, it has to be considered that inhibitory activities were measured at a lower inhibitor concentration than for previous regions. Despite the diversity, there seemed to be only some preference for slightly lipophilic groups in this region, with compounds **(20-23)** demonstrating comparable inhibitory activities. Additionally, disubstitution of the phenyl ring did not lead to an increase in activity in this case **(24, 25).**

**Table 3. In vitro activities for compounds (18-25) in ECF-T whole-cell uptake assay**

| 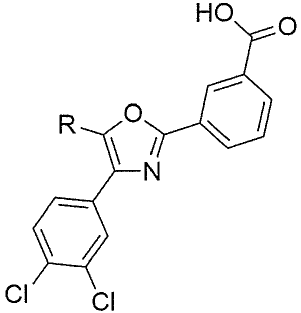 | | | |
|---|---|---|---|
| # | Structure, R= | % inhibition at 25 µM | IC₅₀ (µM) |
| **18** | 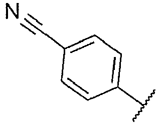 | 85.5 ± 0.8 | n.d. |
| **19** | 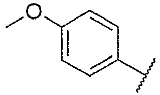 | 98.8 ± 0.3 | 3.5 ± 0.1 |
| **20** | 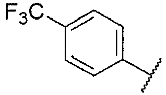 | 99.7 ± 0.1 | 2.5 ± 0.4 |
| **21** | 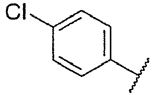 | 96 ± 2.5 | 2.4 ± 0.2 |
| **22** | 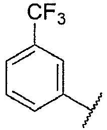 | 99.9 ± 0.1 | 2.2 ± 0.5 |
| **23** | 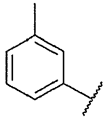 | 99.1 ± 0.1 | 2.4 ± 0.3 |
| **24** | 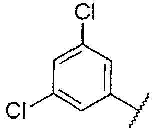 | 89 ± 2.7 | n.d. |
| **25** | 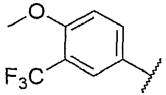 | 98.1 ± 0.3 | 2.6 ± 1.4 |

| | | | |
|---|---|---|---|
| ^{a}Assays were performed in replicate as independent experiments (*n* ≥ 2); values are shown as mean ± SD; n.d. = not determined. | | | |

Compound **22** was used for further exploration of the biological potential of the compounds of the present invention.

### 3. Biological Evaluation

### 3.1. Uptake assay into proteoliposome

To further validate ECF transporters as the target of the compounds of the present invention, a proteoliposome-based uptake assay was performed as demonstrated by Swier et *al*.¹⁸ During this assay, purified folate-specific transporter ECF-FoIT2 from L. *delbrueckii* is reconstituted in proteoliposomes and the uptake of radiolabelled folate into the proteoliposomes is quantified at certain time intervals. As can be seen in Table 4, there is a clear difference in uptake inhibition between **1** and **22.** Whereas **1** demonstrates a relative inhibition of folate uptake of 44% at a concentration 150 µM, **22** reaches a relative inhibition of 95% at the same concentration. In summary, both **1** and **22** are capable of inhibiting the ECF-FoIT2 in a proteoliposome-based uptake assay which further complements the results obtained from the whole-cell uptake assay.

**Table 4. Relative inhibition of ECF-FoIT2 in proteoliposome uptake assay**

| **#** | **Relative inhibition (%)^{a} at 150 µM** |
|---|---|
| **1** | 44 ± 2% |
| **22** | 95 ± 1% |

| | |
|---|---|
| ^{a}Assays were performed in replicate as independent experiments (n = 2); values are shown as mean ± SD. | |

### 2.3. 1. Antimicrobial profiling

Compound **22** was evaluated for its antibacterial activity against a panel of Gram-positive pathogens of relevance. *Enterococcus faecalis* and E. *faecium* are among the most frequent causative agents of hospital-acquired infections, resulting in a wide range of infections, including infections of the urinary tract, bloodstream, and endocardium.¹⁹ *Streptococcus pneumoniae* is the leading cause of pneumonia, especially in elderly and children under five years of age, and poses a great threat to public health in low- and middle-income countries. Furthermore, both *E*. *faecium* and S. *pneumoniae* are on the World Health Organization's priority list for research and development of new antibiotics.²⁰ Compound **22** demonstrated a minimum inhibitory concentration (MIC) of 4 µM against *E. faecalis* and *E. faecium.* Interestingly, the compound exhibited enhanced potency against a vancomycin-resistant strain of *E*. *faecium* (MIC = 2 µM). Even more promising results were obtained for *S*. *pneumoniae,* with 1 demonstrating an MIC between 32 and 16 µM, while **22** achieved the same at a concentration between 0.5 and 1 µM. Additionally, **22** exhibited similar efficacy against a penicillin-resistant strain of *S*. *pneumoniae,* whereas **1** showed reduced potency against this strain. In summary, compound **22** demonstrates significant growth inhibition of relevant strains of Gram-positive bacteria and their resistant variants.

**Table 5. Whole-cell activity of 1 and 22 against relevant Gran-positive pathogens.**

| | **MIC [µM]^{a}** | |
|---|---|---|
| **Strain** | **1** | **22** |
| *Enterococcus faecalis* DSM-20477 | >128 | 4 |
| *E. faecium* DSM-20478 | >128 | 4 |
| *E. faecium* DSM-17050^{b} | >128 | 2 |
| *Streptococcus pneumoniae* DSM-20566 | 32 - 16 | 0.5 - 1 |
| *S*. *pneumoniae* DSM-11865^{c} | >128 | 0.5 - 1 |

| | | |
|---|---|---|
| ^{a}Assays were performed in replicate as independent experiments (n ≥ 2); values are shown as mean ± SD; ^{b}Vancomycin-resistant *Enterococcus faecium* strain; ^{c}Penicillin-resistant *Streptococcus pneumonia* strain; *Enterococcus faecalis;* Minimum inhibitory concentration (MIC). | | |

### 4. Experimental section

### 4.1. General information

Starting materials and solvents were purchased from commercial suppliers, and used without further purification. All chemical yields refer to purified compounds and were not optimized. Column chromatography was performed using the automated flash chromatography system CombiFlash^{®}Rf (Teledyne Isco) equipped with RediSepRf silica columns. Preparative RP-HPLC was performed either using an UltiMate 3000 Semi-Preparative System (Thermo Fisher Scientific) equipped with nucleodur^{®}C18 Gravity (250 mm × 16 mm, 5 µm) column or using a Pure C-850 Flash/Prep (Buchi) equipped with Nucleodur C18 HTec (250 mm × 40 mm, particle size 5 µm). Low-resolution mass spectrometry and purity control of final compounds was carried out using an Ultimate 3000-MSQ LCMS system (Thermo Fisher Scientific) consisting of a pump, an autosampler, an MWD detector and an ESI quadrupole mass spectrometer. ¹H and ¹³C NMR spectra were recorded as indicated on a Bruker Avance Neo 500 MHz (¹H, 500 MHz; ¹³C, 126 MHz) with prodigy cryoprobe system. Chemical shifts were recorded as δ values in ppm units and referenced against the residual solvent peak (DMSO-d6, δ= 2.50, 39.52 and CDCl₃-*d1*: *δ*=7.26, 77.16). Splitting patterns describe apparent multiplicities and are designated as s (singlet), br s (broad singlet), d (doublet), dd (doublet of doublet), t (triplet), q (quartet), m (multiplet). Coupling constants (J) are given in Hertz (Hz). High-resolution mass spectra were recorded on a ThermoFisher Scientific (TF, Dreieich, Germany) Q Exactive Focus system equipped with heated electrospray ionization (HESI)-II source.

### 4.2. Chemistry

The synthesis and analysis of compounds **(3** - **5, 7** - **9, 11** - **15)** was reported in the literature.^{16,17}

### 4.2.1. General procedure 1 (GP-1) for the synthesis of 6 and 10

To a round-bottomed flask, 4-methoxy-4-oxobutanoic acid (1.5 equiv), trimethylamine (2.2 equiv) and acetone were added (100 mL). The solution was stirred at room temperature for 15 min, after which, the respective 2-bromo-1-phenylethan-1-one (1 equiv) was added, and the solution was stirred at room temperature overnight. Subsequently, the solvent was removed *in vacuo,* the residue dissolved in CH₂Cl₂, washed with water (3x), dried over MgSO₄, filtered, and the solvent was removed *in vacuo.* Next, the solid was added to a dry crimp tube, which was charged with acetamide (7 equiv), boron trifluoride diethyl etherate (1.2 equiv) and dry ACN (15 mL). Next, the vial was capped, and the solution was stirred at 120 °C overnight. Afterwards, H₂O (20 mL) was added, and the resulting solution was extracted with a mixture of CHCl₃/propanol (3:1, 3x15 mL). The organic fractions were combined, dried over MgSO₄, filtered, and the solvent removed *in vacuo.* The residue was partially purified using flash chromatography using DCM/MeOH as eluent in a gradient (100% to 95% CH₂Cl₂). The combined fractions were dried, and the solid was added to a flask containing acetic acid (15 mL) and N-bromosuccinimide (1.2 equiv). The resulting solution was stirred at 80 °C for 2 h. Next, water (20 mL) was added, and the mixture was filtered. The collected precipitate was washed with water (3x) and dried at room temperature overnight. The residue was then added to a microwave vial containing phenylboronic acid (1.2 equiv) and CsCOs (2.2 equiv) in a degassed solution of 1,4-dioxane and water (9/1). Next, [Pd(dppf)Cl₂] (0.01 equiv) was added, and the resulting solution was stirred in the microwave at 110 °C for 45 min, after which, more phenylboronic acid (1.2 equiv) was added. The mixture was stirred at 110 °C in the microwave for 45 min. Subsequently, the solution was filtered; to the filtrate, an aqueous solution of LiOH was added (0.5 mL, 2 N), and the resulting solution was stirred at room temperature overnight. Next, the solution was neutralized by the addition of an aqueous solution of HCl (1 N). Water (20 mL) was added, and the resulting mixture was extracted with DCM (3x, 15 mL), dried over MgSO₄, filtered, solvent removed *in vacuo,* and the residue was purified by preparative HPLC affording the title compound.

### 4.2.2. General procedure 2 (GP-2) for the synthesis of 16 and 17

To a round-bottomed flask, the respective carboxylic acid (1 equiv), trimethylamine (2.2 equiv) and acetone were added (100 mL). The solution was stirred at room temperature for 15 min, after which, 2-bromo-1-(3,4-dichlorophenyl)ethan-1-one (1.2 equiv) was added, and the solution was stirred at room temperature overnight. Subsequently, the solvent was removed *in vacuo,* the residue dissolved with CH₂Cl₂, washed with water (3x) dried over MgSO₄, filtered, and the solvent was removed *in vacuo.* Next, the solid was added to a dry crimp tube and acetamide (7 equiv), boron trifluoride diethyl etherate (1.2 equiv) and dry ACN (15 mL) were added. Next, the vial was capped, and the solution was stirred at 120 °C overnight. H₂O (20 mL) was added, and the resulting solution was extracted with a mixture of CHCl₃/propanol (3:1, 3x15 mL). The organic fractions were combined, dried over MgSO₄, filtered, and the solvent removed *in vacuo.* The combined fractions were dried, and the solid was added to a flask containing acetic acid (15 mL) and *N*-bromosuccinimide (1.2 equiv). The resulting solution was stirred at 80 °C for 2 h, after which, water (20 mL) was added, and the mixture was filtered. The collected precipitate was washed with water (3x), and dried at room temperature overnight. The residue was then added to a microwave vial containing phenylboronic acid (1.2 equiv) and CsCOs (2.2 equiv) in a degassed solution of 1,4-dioxane and water (9:1, 5 mL). Next, [Pd(dppf)Cl₂] (0.01 equiv) was added, and the resulting solution was stirred in the microwave at 110 °C for 45 min, after which, more phenylboronic acid (1.2 equiv) was added. The mixture was stirred at 110 °C in the microwave for 45 min. Subsequently, the solution was filtered; to the filtrate, an aqueous solution of LiOH was added (0.5 mL, 2 N), and the resulting solution was stirred at room temperature overnight. Next, the solution was neutralized by the addition of an aqueous solution of HCl (1 N). Water (20 mL) was added, and the resulting mixture was extracted with DCM (3x, 15 mL), dried over MgSO₄, filtered, the solvent removed *in vacuo,* and the residue was purified by preparative HPLC affording the title compound.

### 4.2.3. General procedure 3 (GP-3) for the synthesis of 18 - 25

To a microwave vial containing **II** (1 equiv), Cs₂CO₃ (2.2 equiv) and a degassed solution of 1,4-dioxane/water (9/1, 5 mL), the respective boronic acid was added (1.2 equiv). Next, [Pd(dppf)Cl₂] (0.01 equiv) was added, and the resulting solution was stirred in the microwave at 110 °C for 30 min, after which, more boronic acid (1.2 equiv) was added, and the mixture was again stirred at 110 °C in the microwave for 45 min. Subsequently, the solution was filtered; to the filtrate, an aqueous solution of LiOH was added (0.5 mL, 2 N), and the resulting solution was stirred at room temperature overnight. Next, the solution was neutralized by the addition of an aqueous solution of HCl (1 N). Water (20 mL) was added, and the resulting mixture was extracted with CH₂Cl₂ (3x15 mL), dried over MgSO₄, filtered, and the solvent removed *in vacuo.* The residue was purified by preparative HPLC affording the title compound.

### 4.2.4. 3-(4-(4-Chlorophenyl)-5-phenyloxazol-2-yl)propanoic acid (1)

A flask was charged with succinic acid (0.2 g, 1.7 mmol), trimethylamine (0.5 g, 5.1 mmol) and acetone (20 mL), and the resulting solution was stirred at room temperature for 30 min, after which, 2-bromo-4'-chloroacetophenone (0.4 g, 1.7 mmol) was added. The mixture was stirred at room temperature for 3 h, after which, water (20 mL) was added, and the solution was made slightly basic by the addition of an aqueous solution of NaOH (1N, 2 mL). Next, CH₂Cl₂ (20 mL) was added, and the resulting mixture was extracted (3x), the water fraction was acidified by the addition of an aqueous solution of HCl (1N, 10 mL) and was extracted (3x) by a mixture of CHCl₃/propanol (3:1, 3x15 mL). The resulting organic fractions were washed with saturated aqueous NaCl solution, dried over MgSO₄, filtered, and solvent was removed *in vacuo.* The residue was then added to a crimp vail containing acetamide (0.5 g, 8.6 mmol) and boron trifluoride diethyl etherate (0.2 g, 1.7 mmol). The tube was capped and mixed under neat conditions overnight at 100 °C. Next, water was added, and the solution was made slightly basic by the addition of an aqueous solution of NaOH (1N, 2 mL). Next CH₂Cl₂ was added, and the resulting mixture was extracted (3x), the water fraction was acidified by the addition of an aqueous solution of HCl (1 N, 10 mL) and was extracted with a mixture of CHCl₃/propanol (3:1, 3x15 mL). The resulting organic fractions were washed with saturated aqueous NaCl solution, dried over MgSO₄, filtered, and the solvent was removed *in vacuo.* Next, chloroform (3 mL) and N-bromosuccinimide (0.1 g, 0.6 mmol) were added, and the resulting mixture was stirred at reflux for 2 h after which, water was added. The resulting mixture was made slightly basic by the addition of an aqueous solution of NaOH (1 N, 2 mL). Next, EtOAc was added, and the resulting mixture was extracted (3x), the water fraction was acidified by the addition of an aqueous solution of HCl (1 N, 10 mL) and was extracted (3x) by a mixture of CHCl₃/propanol (3:1, 3x15 mL). The resulting organic fractions were washed with saturated aqueous NaCl solution, dried over MgSO₄, filtered, and the solvent was removed *in vacuo.* The solid was added to a degassed solution of 1,4-dioxane/H₂O (4:1, 20 mL) containing Na₂CO₃ (0.1 g, 1.3 mmol) and phenylboronic acid (0.1 g, 0.4 mmol). Polymer-bound [Pd[(C₆H₅)₃P]₄] (0.01 g) was added, and the reaction mixture was stirred overnight at 100 °C. Next, an aqueous solution of HCl (1 N, 25 mL) was added, and the resulting mixture was extracted (3x) with a mixture of CHCl₃/propanol (3:1, 3x15 mL). The combined organic fractions were washed with saturated aqueous NaCl solution, dried over MgSO₄, filtered, and dried *in vacuo.* The residue was purified using preparative RP-HPLC, affording **1** as a white solid (0.01 g, 2% yield). ¹H NMR (500 MHz, DMSO-d6) δ = 12.35 (br s, 1H), 7.88 (d, *J* = 8.5 Hz, 1H), 7.56 (d, *J* = 8.5 Hz, 1H), 7.58 - 7.54 (m, 1H), 3.04 - 2.99 (m, 1H), 2.76 - 2.72 (m, 1H). ¹³C NMR (126 MHz, DMSO-d6) *δ* = 173.5, 163, 145.3, 133.5, 133, 131.2, 129.4, 129.3, 129.1, 128.5, 126.9, 30.6, 23.3. HRMS (ESI+) calculated for C₁₈H₁₅ClNO₃ [M+H]⁺ 328.06622, found 328.07302. Purity by HPLC = 100%.

### 4.2.4. 3-(5-(4-Cyanophenyl)-4-phenyloxazol-2-yl)propanoic acid. (6).

According to GP-1, using 4-(2-bromoacetyl)benzonitrile (0.4 g, 1.79 mmol) afforded after purification by preparative HPLC **6** as a white solid (0.01g, 2% yield). ¹H NMR (500 MHz, CDCl₃-*d*) *δ* = 7.77 (d, *J* = 8.2 Hz, 2H), 7.63 (d, *J* = 8.2 Hz, 2H), 7.57 - 7.52 (m, 2H), 7.44 - 7.39 (m, 3H), 3.25 - 3.16 (m, 2H), 3.03 - 2.93 (m, 2H). ¹³C NMR (126 MHz, CDCl₃-*d*) δ = 176.5, 162.5, 147.6, 137, 133.5, 132.6, 129.7, 129.2, 128.4, 128.3, 127.3, 119, 111.6, 30.8, 23.4. HRMS (ESI+) calculated for C₁₉H₁₅N₂O₃ [M+H]⁺ 319.10044, found 319.10768. Purity by HPLC = 100%.

### 4.2.5. 3-(4-(3,5-Dichlorophenyl)-5-phenyloxazol-2-yl)propanoic acid (10).

According to GP-1, using 2-bromo-1-(3,5-dichlorophenyl)ethan-1-one (0.45 g, 1.68 mmol), affording after purification by preparative HPLC **10** as a white solid (0.01g, 2% yield). ¹H NMR (500 MHz, CDCl₃-*d*) *δ* = 7.56 (d, *J* = 1.8 Hz, 1H), 7.55 - 7.53 (m, 3H), 7.44 - 7.39 (m, 3H), 7.31 (t, *J* = 1.8 Hz, 1H), 3.24 - 3.16 (m, 2H), 3.02 - 2.95 (m, 2H). ¹³C NMR (126 MHz, CDCl₃-*d*) *δ* = 176.9, 162.2, 147, 135.4, 132.7, 129.6, 129.2, 128.3, 127.1, 126.2, 30.8, 23.4. HRMS (ESI+) calculated for C₁₈H₁₄Cl₂NO₃ [M+H]⁺ 362.02725, found 362.01485. Purity by HPLC = 99%.

### 4.2.6. 2-(4-(3,4-Dichlorophenyl)-5-phenyloxazol-2-yl)benzoic acid (16)

According to GP-2, using 2-(methoxycarbonyl)benzoic acid (0.4 g, 1.49 mmol), affording after purification by preparative HPLC **16** as a white solid (0.01 g, 2% yield). ¹H NMR (500 MHz, CDCl₃-*d*) δ = 8.48 (dd, *J* = 7.7, 1.0 Hz, 1H), 8.23 (dd, *J* = 7.8, 1.1 Hz, 1H), 7.80 (d, *J* = 1.8 Hz, 1H), 7.74 - 7.70 (m, 1H), 7.69 - 7.63 (m, 3H), 7.62 - 7.62 (m, 1H), 7.54 - 7.50 (m, 1H), 7.49 - 7.45 (m, 4H). ¹³C NMR (126 MHz, CDCl₃-*d*) *δ* = 167.3, 159.6, 147.3, 134.5, 133.3, 133.1, 132.4, 132.4-132.3 (m, 1C), 131.5, 130.9, 130.4, 130.3, 130.2, 129.4, 129.3, 129.2, 127.1, 127, 126.8, 123.9. HRMS (ESI+) calculated for C₂₂H₁₄Cl₂NO₃ [M+H]⁺ 410.02725, found 410.003459. Purity by HPLC: 100%.

### 4.2.7. 4-(4-(3,4-Dichlorophenyl)-5-phenyloxazol-2-yl)picolinic acid (17)

According to GP-2, using 2-(methoxycarbonyl)isonicotinic acid (0.5 g, 2.76 mmol), affording after purification by preparative HPLC **17** as a yellowish solid (0.1 g, 1% yield). ¹H NMR (500 MHz, CDCl₃-d) δ = 8.91 - 8.87 (m, 1H), 8.79 (br d, *J* = 4.9 Hz, 1H), 8.29 (br d, J = 4.4 Hz, 1H), 7.90 (d, J = 1.2 Hz, 1H), 7.72 - 7.65 (m, 2H), 7.56 (dd, J = 8.3, 1.4 Hz, 1H), 7.53 - 7.45 (m, 4H). ¹³C NMR (126 MHz, CDCl₃-*d*) *δ* = 163.7, 156.8, 149, 136.6, 135.5, 133.1, 132.8, 131.6, 130.7, 130.1, 129.8, 129.2, 127.4, 127.2, 126.9, 123.9, 120.1. HRMS (ESI+) calculated for C₂₁H₁₃Cl₂N₂O₃ [M+H]⁺ 411.02250, found 411.03005. Purity by HPLC: 100%.

### 4.2.8. 3-(5-(4-Cyanophenyl)-4-(3,4-dichlorophenyl)oxazol-2-yl)benzoic acid (18).

According to GP-3, using (4-cyanophenyl)boronic acid (0.02g, 0.14 mmol), affording after purification by preparative HPLC **18** as a white solid (0.01 g, 15% yield). ¹H NMR (500 MHz, DMSO-d6) δ = 8.65 - 8.62 (m, 1H), 8.38 (d, *J* = 7.8 Hz, 1H), 8.14 (d, *J =* 7.6 Hz, 1H), 7.99 - 7.96 (m, 2H), 7.93 (d, *J* = 1.8 Hz, 1H), 7.89 (d, *J* = 8.2 Hz, 2H), 7.78 - 7.71 (m, 2H), 7.63 (dd, *J* = 8.4, 1.7 Hz, 1H). ¹³C NMR (126 MHz, DMSO-d6) δ = 166.2, 159.5, 144.5, 135.8, 132.8, 131.6, 131.6, 131.5, 131.4, 131.3, 131, 129.4, 127.7, 126.8, 126.6, 118.1, 111.1, 54.6. HRMS (ESI+) calculated for C₂₃H₁₃Cl₂N₂O₃ [M+H]⁺ 435.02250, found 435.02912. Purity by HPLC: 98%.

### 4.2.9. 3-(4-(3,4-Dichlorophenyl)-5-(4-methoxyphenyl)oxazol-2-yl)benzoic acid (19).

According to GP-3, using (4-methoxyphenyl)boronic acid (0.03 g, 0.21 mmol), affording after purification by preparative HPLC **19** as a white solid (0.1 g, 27% yield). ¹H NMR (500 MHz, DMSO-d6) δ = 8.59 (s, 1H), 8.32 (d, *J* = 7.8 Hz, 1H), 8.11 (d, *J* = 7.8 Hz, 1H), 7.89 (d, *J* = 2.0 Hz, 1H), 7.74 - 7.69 (m, 2H), 7.64 (d, *J* = 8.7 Hz, 2H), 7.63 - 7.60 (m, 1H), 7.10 (d, *J* = 8.9 Hz, 2H), 3.83 (s, 3H). ¹³C NMR (126 MHz, DMSO-d6) *δ* = 167.3-166.9 (m, 1C), 159, 147.4, 133, 132.1, 131.9, 131.6, 131.2, 130.6, 130.3, 129.4, 127.7, 127.2, 127.1, 120.2, 115.2. HRMS (ESI+) calculated for C₂₃H₁₆Cl₂NO₄ [M+H]⁺ 440.03781, found 440.04358. Purity by HPLC: 100%.

### 4.2.10. 3-(4-(3,4-Dichlorophenyl)-5-(4-(trifluoromethyl)phenyl)oxazol-2-yl)benzoic acid (20).

According to GP-3, using (4-(trifluoromethyl)phenyl)boronic acid (0.03g, 0.14 mmol), affording after purification by preparative HPLC **20** as a white solid (0.01 g, 8% yield). ¹H NMR (500 MHz, DMSO-d6) *δ* = 8.63 (s, 1H), 8.34 (br d, *J* = 7.6 Hz, 1H), 8.13 (d, *J* = 7.8 Hz, 1H), 7.94 - 7.90 (m, 3H), 7.90 - 7.86 (m, 2H), 7.76 (d, *J* = 8.4 Hz, 1H), 7.73 - 7.69 (m, 1H), 7.64 (dd, *J* = 8.4, 1.8 Hz, 1H). ¹³C NMR (126 MHz, DMSO-d6) *δ* = 166.8, 160, 135.7, 132.2, 132, 131.9, 131.7, 131.6, 131.4, 129.8, 129.8, 128.1, 127.5, 127.1, 126.4, 126.3, 55.1. ¹⁹F NMR (470 MHz, DMSO-d6) *δ* = -61.3 (s). HRMS (ESI+) calculated for C₂₃H₁₃Cl₂F₃NO₃ [M+H]⁺ 478.01463, found 478.02262. Purity by HPLC: 98%.

### 4.2.11. 3-(5-(4-Chlorophenyl)-4-(3,4-dichlorophenyl)oxazol-2-yl)benzoic acid (21).

According to GP-3, using (4-chlorophenyl)boronic acid (0.03 g, 0.21 mmol), affording after purification by preparative HPLC **21** as a white solid (0.01 g, 12% yield). ¹H NMR (500 MHz, DMSO-d6) δ = 8.62 - 8.60 (m, 1H), 8.35 - 8.32 (m, 1H), 8.14 - 8.10 (m, 1H), 7.91 - 7.89 (m, 1H), 7.75 - 7.70 (m, 4H), 7.63 - 7.58 (m, 3H). ¹³C NMR (126 MHz, DMSO-d6) δ = 167.1, 159.7, 146, 134.8, 134.7, 132.6, 132.2, 132.1, 131.7, 131.7, 130.6, 130.2, 129.8, 129.8, 129.2, 128.1, 127.2, 126.9, 126.8. HRMS (ESI+) calculated for C₂₂H₁₃Cl₃NO₃ [M+H]⁺ 443.98828, found 443.99533. Purity by HPLC: 95%.

### 4.2.12. 3-(4-(3,4-Dichlorophenyl)-5-(3-(trifluoromethyl)phenyl)oxazol-2-yl)benzoic acid (22).

According to GP-3, using (3-(trifluoromethyl)phenyl)boronic acid (0.03 g, 0.14 mmol), affording after purification by preparative HPLC 22 as a white solid (0.02 g, 35% yield). ¹H NMR (500 MHz, DMSO-*d6*) δ = 8.63 (s, 1H), 8.39 (d, *J* = 7.8 Hz, 1H), 8.13 (d, *J =* 7.8 Hz, 1H), 8.04 (s, 1H), 7.99 (d, *J* = 7.8 Hz, 1H), 7.91 (d, *J* = 1.8 Hz, 1H), 7.86 (d, *J* = 7.9 Hz, 1H), 7.78 - 7.71 (m, 3H), 7.63 (dd, *J* = 8.4, 1.8 Hz, 1H). ¹³C NMR (126 MHz, DMSO-d6) δ = 166.2, 159.2, 144.7, 134.6, 131.6, 131.4, 131.4, 131, 130.9, 130.5, 130.1, 130.1, 129.5, 128.9, 128.2, 127.4, 126.6, 126.1, 54.6. ¹⁹F NMR (470 MHz, DMSO-*d6*) *δ* = -61.39 (s). HRMS (ESI+) calculated for C₂₃H₁₃Cl₂F₃NO₃ [M+H]⁺ 478.01463, found 478.02030. Purity by HPLC: 100%.

### 4.2.13. 3-(4-(3,4-Dichlorophenyl)-5-(m-tolyl)oxazol-2-yl)benzoic acid (23).

According to GP-3, using *m*-tolylboronic acid (0.02 g, 0.14 mmol), affording after purification by preparative HPLC **23** as a white solid (0.02 g, 30% yield). ¹H NMR (500 MHz, DMSO-d6) *δ* = 8.60 (s, 1H), 8.34 (d, *J* = 7.8 Hz, 1H), 8.12 (d, *J* = 7.8 Hz, 1H), 7.90 (d, *J* = 2.0 Hz, 1H), 7.74 - 7.70 (m, 2H), 7.63 (dd, *J* = 8.5, 1.9 Hz, 1H), 7.57 (s, 1H), 7.48 - 7.45 (m, 1H), 7.43 - 7.39 (m, 1H), 7.34 - 7.31 (m, 1H), 2.39 - 2.36 (m, 3H). ¹³C NMR (126 MHz, DMSO-d6) *δ* = 166.6, 158.9, 146.8, 138.7, 133.6, 132.4, 131.5, 131.1, 130.9, 130.5, 130.2, 129.8, 129.1, 129, 127.5, 127.4, 127.4, 126.7, 126.6, 124.3, 54.9, 20.9 HRMS (ESI+) calculated for C₂₃H₁₆Cl₂NO₃ [M+H]⁺ 424.04290, found 424.04891. Purity by HPLC: 99%.

### 4.2.14. 3-(4-(3, 4-Dichlorophenyl)-5-(3, 5-dichlorophenyl)oxazol-2-yl)benzoic acid (24).

According to GP-3, using 3,5-dichlorophenyl)boronic acid (0.03 g, 0.14 mmol), affording after purification by preparative HPLC **24** as a white solid (0.01 g, 4% yield).

¹H NMR (500 MHz, DMSO-d6) *δ* =13.40 (br s, 1H), 8.64 (s, 1H), 8.41 (brd, *J* = 7.6 Hz, 1H), 8.14 (br d, *J* = 7.8 Hz, 1H), 7.96 - 7.90 (m, 1H), 7.80 - 7.69 (m, 5H), 7.69 - 7.58 (m, 1H), 7.58 - 7.58 (m, 1H). ¹³C NMR (126 MHz, DMSO-d6) *δ* = 167.1, 160.2 - 160.1 (m, 1C), 144.2, 136.1, 135.4, 132.4, 132.2, 132.2, 132, 131.7, 131.3, 131, 130.3, 130, 129.4, 128.3, 127.5, 126.8, 125.8. HRMS (ESI+) calculated for C₂₂H₁₂Cl₄NO₃ [M+H]⁺ 479.94635, found: 479.95290. Purity by HPLC: 100%.

### 4.2.15. 3-(4-(3,4-Dichlorophenyl)-5-(4-methoxy-3-(trifluoromethyl)phenyl)oxazol-2-yl)benzoic acid (25).

According to GP-3, using (4-methoxy-3-(trifluoromethyl)phenyl)boronic acid (0.03 g, 0.14 mmol), affording after purification by preparative HPLC **25** as a white solid (0.03 g, 57% yield). ¹H NMR (500 MHz, DMSO-d6) δ = 8.61 (s, 1H), 8.35 (d, *J* = 7.8 Hz, 1H), 8.12 (d, *J* = 7.8 Hz, 1H), 7.96 - 7.88 (m, 3H), 7.74 - 7.69 (m, 2H), 7.61 (dd, *J* = 8.3, 1.9 Hz, 1H), 7.43 (d, *J* = 8.7 Hz, 1H), 3.97 (s, 3H) ¹³C NMR (126 MHz, DMSO-d6) *δ* = 166.8, 159.1, 158, 145.6, 133.7, 133.3, 132.3, 132.1, 131.8, 131.7, 131.3, 131.2, 130.4, 130, 129.2, 127.6, 127.6, 126.9, 126.7, 125.8, 125.7, 119.8, 114, 56.7, 55.1. ¹⁹F NMR (470 MHz, DMSO-d6) δ = -61.25 (s). HRMS (ESI+) calculated for C₂₄H₁₅Cl₂F₃NO₄ [M+H]⁺ 508.02520, found 508.03169. Purity by HPLC: 98%.

### 4.3. Biological assays

### 4.3.1. ECF-FoIT inhibition assay.

This assay was performed based on the protocol reported by Bousis *et al.* with minor modifications.²²

### 4.3.2. Uptake assay into proteoliposome

This assay was performed based on the protocol reported by Swier *et al.* with minor modifications.¹⁸

### 4.3.3. Screening against clinically relevant Gram-positive pathogens.

All microorganisms were obtained from the German Collection of Microorganisms and Cell Cultures (DSMZ) and were handled according to standard procedures. Bacteria were inoculated into tryptic soy broth (TSB) to obtain a final inoculum of 105 colony-forming units (CFU)/mL. The tested compounds were prepared as DMSO stocks (20 mM). Serial dilutions of derivatives in the growth medium (0.06 to 128 µM) were prepared in sterile 96-well plates, and the bacterial suspensions were added. Growth inhibition was assessed after static incubation at 37 °C for 24 h. S. *pneumoniae* was grown at 5% CO₂. MIC values are defined as the lowest compound concentration where no visible growth is observed.

### 5. Further compounds

| **Code** | **Structure** | % inhibition at 50 µM | **Code** | **Structure** | % inhibition at 50 µM |
|---|---|---|---|---|---|
| **26** | 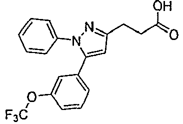 | 21.1 ± 6.4 | **32** | 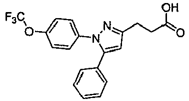 | 23.7 ± 7.7 |
| **27** | 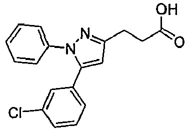 | 19.9 ± 3.3 | **33** | 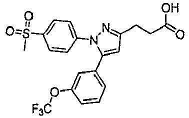 | 5.3 ± 1.2 |
| **28** | 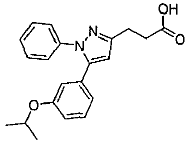 | 12.2 ± 9.8 | **34** | 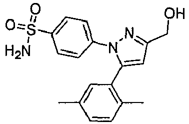 | 7.7 ± 3.9 |
| **29** | 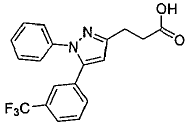 | 10.2 ± 11.0 | **35** | 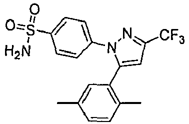 | 21.2 4.9 |
| **30** | 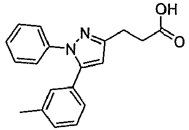 | 7.4 ± 0.9 | **36** | 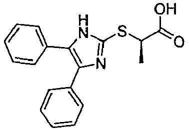 | 16.8 ± 1.0 |
| **31** | 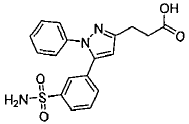 | 7.4 ± 1.6 | | | |

### General Scheme²² for compounds 26-35:

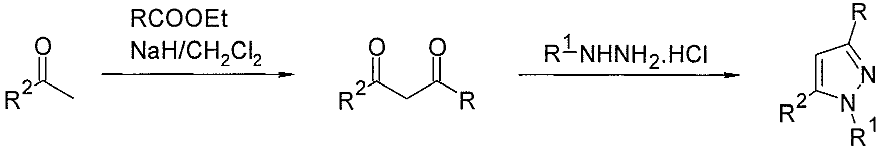

### Example compound 35:

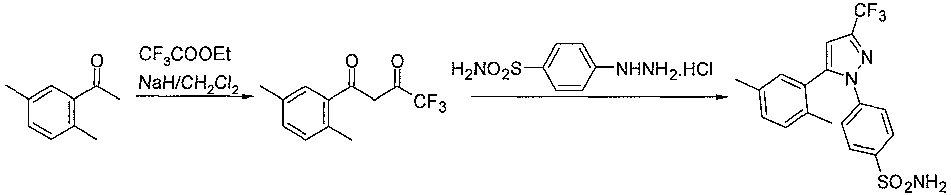

Compound 36 is commercially available and can be synthesized according to the general reported procedures^{23-2a}.

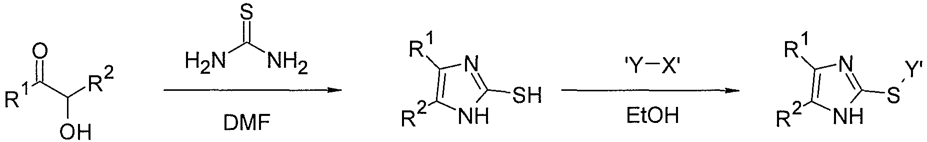

### References

(1) Henderson, G. B.; Zevely, E. M. Binding and transport of thiamine by Lactobacillus casei. J Bacteriol 1978, 133 (3), 1190-1196. DOI: 10.1128/jb.133.3.1190-1196.1978. Henderson, G. B.; Zevely, E. M.; Huennekens, F. M. Coupling of energy to folate transport in Lactobacillus casei. J Bacteriol 1979, 139 (2), 552-559. DOI: 10.1128/jb.139.2.552-559.1979. Henderson, G. B.; Zevely, E. M.; Kadner, R. J.; Huennekens, F. M. The folate and thiamine transport proteins of Lactobacillus casei. J Supramol Struct 1977, 6 (2), 239-247. DOI: 10.1002/jss.400060209.
(2) Slotboom, D. J. Structural and mechanistic insights into prokaryotic energy-coupling factor transporters. Nat Rev Microbiol 2014, 12 (2), 79-87. DOI: 10.1038/nrmicro3175 From NLM Medline.
(3) Eudes, A.; Erkens, G. B.; Slotboom, D. J.; Rodionov, D. A.; Naponelli, V.; Hanson, A. D. Identification of genes encoding the folate- and thiamine-binding membrane proteins in Firmicutes. J Bacteriol 2008, 190 (22), 7591-7594. DOI: 10.1128/JB.01070-08.
(4) Song, J.; Ji, C.; Zhang, J. Z. Unveiling the gating mechanism of ECF transporter RibU. Sci Rep 2013, 3, 3566. DOI: 10.1038/srep03566.
(5) Santos, J. A.; Rempel, S.; Mous, S. T.; Pereira, C. T.; Ter Beek, J.; de Gier, J. W.; Guskov, A.; Slotboom, D. J. Functional and structural characterization of an ECF-type ABC transporter for vitamin B12. Elife 2018, 7. DOI: 10.7554/eLife.35828.
(6) Finkenwirth, F.; Kirsch, F.; Eitinger, T. Solitary BioY proteins mediate biotin transport into recombinant Escherichia coli. J Bacteriol 2013, 195 (18), 4105-4111. DOI: 10.1128/JB.00350-13.
(7) Majsnerowska, M.; Ter Beek, J.; Stanek, W. K.; Duurkens, R. H.; Slotboom, D. J. Competition between Different S-Components for the Shared Energy Coupling Factor Module in Energy Coupling Factor Transporters. Biochemistry 2015, 54 (31), 4763-4766. DOI: 10.1021/acs.biochem.5b00609.
(8) Erkens, G. B.; Berntsson, R. P.; Fulyani, F.; Majsnerowska, M.; Vujicic-Zagar, A.; Ter Beek, J.; Poolman, B.; Slotboom, D. J. The structural basis of modularity in ECF-type ABC transporters. Nat Struct Mol Biol 2011, 18 (7), 755-760. DOI: 10.1038/nsmb.2073.
(9) Zhang, M.; Bao, Z.; Zhao, Q.; Guo, H.; Xu, K.; Wang, C.; Zhang, P. Structure of a pantothenate transporter and implications for ECF module sharing and energy coupling of group II ECF transporters. Proc Natl Acad Sci U S A 2014, 111 (52), 18560-18565.
(10) Yu, Y.; Zhou, M.; Kirsch, F.; Xu, C.; Zhang, L.; Wang, Y.; Jiang, Z.; Wang, N.; Li, J.; Eitinger, T.; et al. Planar substrate-binding site dictates the specificity of ECF-type nickel/cobalt transporters. Cell Res 2014, 24 (3), 267-277. DOI: 10.1038/cr.2013.172. Bao, Z.; Qi, X.; Hong, S.; Xu, K.; He, F.; Zhang, M.; Chen, J.; Chao, D.; Zhao, W.; Li, D.; et al. Structure and mechanism of a group-I cobalt energy coupling factor transporter. Cell Res 2017, 27 (5), 675-687. DOI: 10.1038/cr.2017.38.
(11) Bousis, S.; Setyawati, I.; Diamanti, E.; Slotboom, D. J.; Hirsch, A. K. H. Energy-Coupling Factor Transporters as Novel Antimicrobial Targets. Advanced Therapeutics 2019, 2(2).
(12) Davidson, A. L.; Dassa, E.; Orelle, C.; Chen, J. Structure, function, and evolution of bacterial ATP-binding cassette systems. Microbiol Mol Biol Rev 2008, 72 (2), 317-364, table of contents. DOI: 10.1128/MMBR.00031-07.
(13) Rodionov, D. A.; Hebbeln, P.; Eudes, A.; ter Beek, J.; Rodionova, I. A.; Erkens, G. B.; Slotboom, D. J.; Gelfand, M. S.; Osterman, A. L.; Hanson, A. D.; et al. A novel class of modular transporters for vitamins in prokaryotes. J Bacteriol 2009, 191 (1), 42-51.
(14) Berntsson, R. P.; Smits, S. H.; Schmitt, L.; Slotboom, D. J.; Poolman, B. A structural classification of substrate-binding proteins. FEBS Left 2010, 584 (12), 2606-2617. DOI: 10.1016/j.febslet.2010.04.043.
(15) ter Beek, J.; Duurkens, R. H.; Erkens, G. B.; Slotboom, D. J. Quaternary structure and functional unit of energy coupling factor (ECF)-type transporters. J Biol Chem 2011, 286 (7), 5471-5475. DOI: 10.1074/jbc.M110.199224.
(16) Kramer JS, Woltersdorf S, Duflot T, Hiesinger K, Lillich FF, Knöll F, et al. Discovery of the First in Vivo Active Inhibitors of the Soluble Epoxide Hydrolase Phosphatase Domain. Journal of Medicinal Chemistry 2019; 62, 8443-60.
(17) Schierle S, Chaikuad A, Lillich FF, Ni X, Woltersdorf S, Schallmayer E, et al. Oxaprozin Analogues as Selective RXR Agonists with Superior Properties and Pharmacokinetics. Journal of Medicinal Chemistry 2021; 64, 5123-36.
(18) Swier LJYM, Guskov A, Slotboom DJ. Structural insight in the toppling mechanism of an energy-coupling factor transporter. Nature Communications 2016; 7, 11072.
(19) Brinkwirth S, Ayobami O, Eckmanns T, Markwart R. Hospital-acquired infections caused by enterococci: a systematic review and meta-analysis, WHO European Region, 1 January 2010 to 4 February 2020. Eurosurveillance 2021; 26, 2001628.
(20) Organization GWH. Prioritization of pathogens to guide discovery, research and development of new antibiotics for drug-resistant bacterial infections, including tuberculosis. 2017**.**
(21) Bousis S, Winkler S, Haupenthal J, Fulco F, Diamanti E, Hirsch AKH. An Efficient Way to Screen Inhibitors of Energy-Coupling Factor (ECF) Transporters in a Bacterial Uptake Assay. International Journal of Molecular Sciences 2022**.**
(22) Chen, C. S., Song, X., & Lin, H. P. Compounds and methods for inducing apoptosis in proliferating cells. U.S. Patent No. 7,026,346. 11 Apr. 2006**.**
(23) Sharpe, T. R., Cherkofsky, S. C., Hewes, W. E., Smith, D. H., Gregory, W. A., Haber, S. B., Leadbetter, M.R., Whitney, J. G. Preparation, antiarthritic and analgesic activity of 4, 5-diaryl-2-(substituted thio)-1H-imidazoles and their sulfoxides and sulfones. Journal of medicinal chemistry 1985; 28(9), 1188-1194.
(24) Ali, M. I., Abou-State, M. A., & Ibrahim, A. F. Reactions with 5, 6-Diphenyl-2, 3-dihydro-imidazo [2, 1-b] thiazol-3-ones and 6, 7-Diphenyl-2, 3-dihydro-4H-imidazo [2, 1-b] 1, 3-thiazin-4-one. Journal für Praktische Chemie 1974; 316(1), 147-153.

## Claims

1. A compound of formula (Ia) or (Ib): 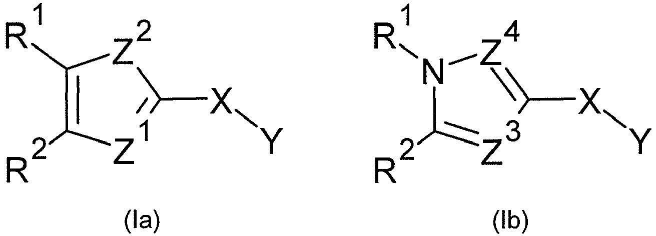 wherein
Z¹ N or CH;
Z² is O, S or NH;
Z³ is N or CH;
Z⁴ is N or CH;
Y is hydrogen; halogen; -C₁₋₄ alkyl; -C₁₋₄ haloalkyl; COOH; OH; CONH₂; CONHR'; PO(OH)₂; or PO(OR')₂;
R' is a C₁₋₄ alkyl group;
X is a bond; a C₁₋₄ alkylene group; a -S-C₁₋₄ alkylene group; a -O-C₁₋₄ alkylene group; a -NH-C₁₋₄ alkylene group; an optionally substituted phenylene group; or an optionally substituted heteroarylene group containing 5 or 6 ring atoms that are independently selected from C, N, O and S;
R¹ is hydrogen, halogen, an alkyl group, a cycloalkyl group, an optionally substituted phenyl group; or an optionally substituted heteroaryl group containing 5 or 6 ring atoms that are independently selected from C, N, O and S; and
R² is hydrogen, halogen, an alkyl group, a cycloalkyl group, an optionally substituted phenyl group; or an optionally substituted heteroaryl group containing 5 or 6 ring atoms that are independently selected from C, N, O and S;
or a salt thereof.

2. A compound according to claim 1 of formula (Ila) or (lib), or a salt thereof:

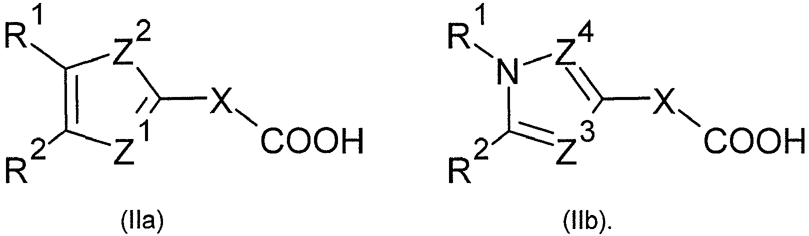

3. A compound according to claim 1 or 2 of formula (III), or a salt thereof:

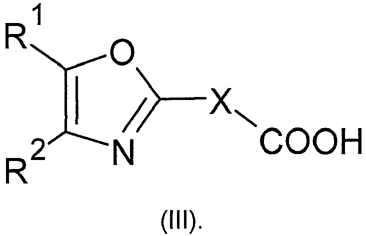

4. A compound according to any one of the preceding claims, wherein
X is a C₁₋₄ alkylene group; an optionally substituted phenylene group; or an optionally substituted heteroarylene group containing 5 or 6 ring atoms that are independently selected from C, N, O and S;
R¹ is an optionally substituted phenyl group; or an optionally substituted heteroaryl group containing 5 or 6 ring atoms that are independently selected from C, N, O and S; and
R² is an optionally substituted phenyl group; or an optionally substituted heteroaryl group containing 5 or 6 ring atoms that are independently selected from C, N, O and S.

5. A compound according to any one of the preceding claims, wherein X is selected from the following groups: -CH₂-CH₂-; -CH₂-CH₂-CH₂-;

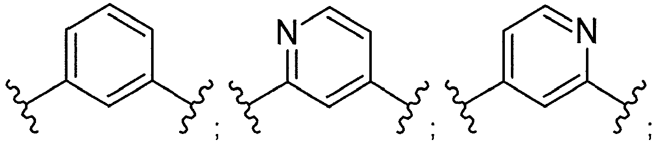

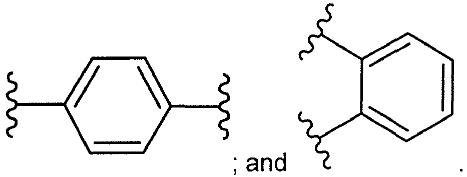

6. A compound according to any one of the preceding claims, wherein X is selected from the following groups:

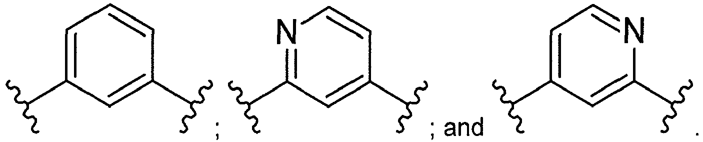

7. A compound according to any one of the preceding claims, wherein X is the following group:

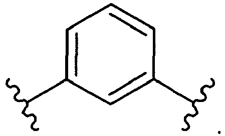

8. A compound according to any one of the preceding claims, wherein R¹ is an optionally substituted phenyl group.

9. A compound according to any one of the preceding claims, wherein R² is an optionally substituted phenyl group.

10. A compound according to any one of the preceding claims, wherein R¹ and/or R² are unsubstituted or substituted by one or two (especially by one) substituent(s).

11. A compound according to any one of the preceding claims, wherein the optional substituents are independently selected from halogen; -OH; -NH₂; -CN; -C₁₋₄ alkyl; -C₁₋₄ haloalkyl; -O-C₁₋₄ alkyl; -O-C₁₋₄ haloalkyl; -SO₂NH₂; -SO₂CH₃; -O-CH₂-CH₂-; and -O-CH₂-O-.

12. A compound according to any one of the preceding claims, wherein the optional substituents are independently selected from -F; -Cl; -Me; -OMe; -CF₃; -OCF₃; -SO₂NH₂; -SO₂CH₃; -OCH(CH₃)₂; and -CN.

13. A compound selected from the following compounds: 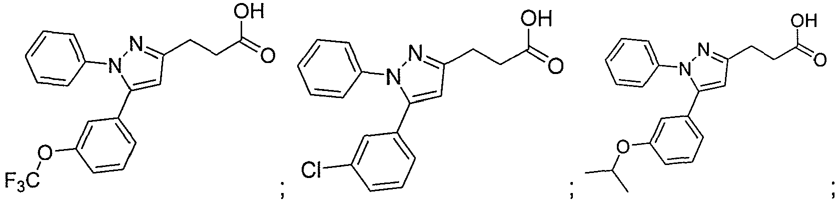 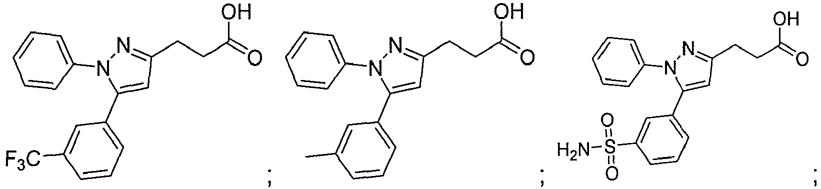 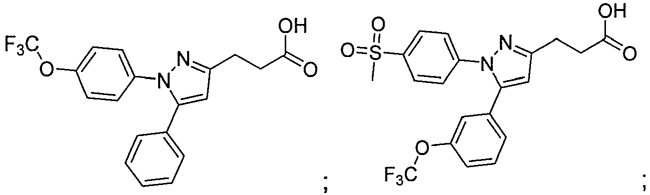 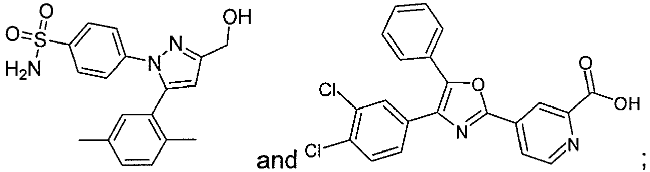 and the following compounds: 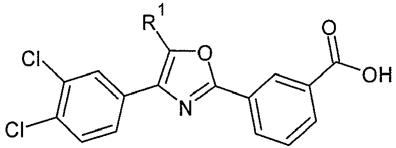 wherein R¹ is selected from the following groups:

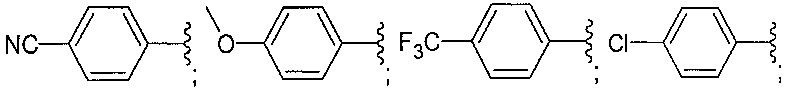

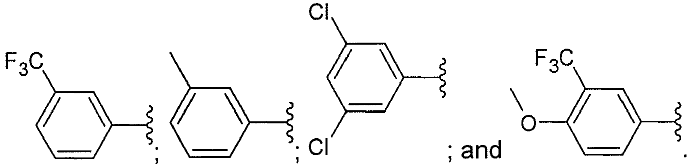

14. Pharmaceutical composition comprising a compound according to anyone of the preceding claims and optionally one or more carrier substances and/or one or more adjuvants.

15. Compound according to any one of claims 1 to 13 or pharmaceutical composition according to claim 14 for use in the treatment of bacterial infections, especially caused by *Streptococcus pneumoniae, Enterococcus faecium, Enterococcus faecalis.*
